# EUROPEAN PATENT APPLICATION

(11) **EP 0 983 997 A1**
(43) Date of publication of application: **08.03.2000**
(21) Application number: 98909780.3
(22) Date of filing: 20.03.1998
(51) Int. Cl.: C07C 255/14, C07D 277/30, C08F 20/42, A01N 37/34, A01N 43/78

(54) **RESINS FOR ANTIFOULING AGENTS AGAINST PERIPHYTONS, PROCESS FOR THE PREPARATION OF THE RESINS, AND ANTIFOULING AGENTS AGAINST PERIPHYTONS CONTAINING THE SAME**

(71) Applicant: IHARA CHEMICAL INDUSTRY CO., LTD., Taitoh-ku, Tokyo 110-0008 (JP)
(72) Inventor: OHASHI, Hideaki, Ihara Chemical Industry Co., Ltd., Ihara-gun, Shizuoka-ken 421-3306 (JP)
(74) Representative: Laufhütte, Dieter, Dr.-Ing.
(86) International application number: JP9801200
(87) International publication number: WO9948862

(57) **Abstract**

The present invention provides:
a compound used for production of a resin used in an anti-fouling agent for aquatic fouling organisms, said compound being represented by the following general formula (1): 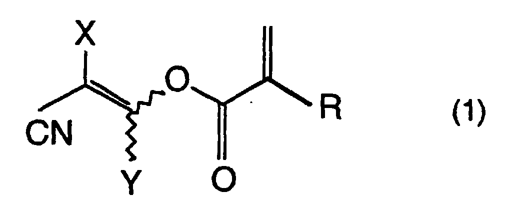
a resin used in an anti-fouling agent for aquatic fouling organisms, which is obtained by polymerizing at least one kind of compound mentioned above and at least one kind of unsaturated monomer selected from other unsaturated monomers having radical-polymerizable double bond,
a process for producing a resin used in an anti-fouling agent for aquatic fouling organisms, which is characterized by polymerizing at least one kind of compound mentioned above and at least one kind of unsaturated monomer selected from other unsaturated monomers having radical-polymerizable double bond, and
an anti-fouling agent for aquatic fouling organisms, which is characterized by containing a resin mentioned above.

## Description

### Technical Field

The present invention relates to a resin used in an anti-fouling agent for aquatic fouling organisms, used for prevention of attachment of aquatic organisms onto underwater construction, fishing net, ship bottom, etc.; a process for production of said resin; a compound used in production of said resin; and an anti-fouling agent for aquatic fouling organisms, using said resin.

### Background Art

As the resin for use in anti-fouling agent for aquatic fouling organisms, there are known so-called organotin copolymers which are gradually hydrolyzed in sea water to generate an acrylic resin soluble in sea water and an organotin compound (an effective component). Anti-fouling paints using such an organotin copolymer are excellent in prevention of the attachment of aquatic organisms because the coating film produced from such a paint is polished in sea water and releases the organotin copolymer contained in the film and this organotin copolymer, when hydrolyzed, generates an organotin compound of high anti-fouling activity. The use of the anti-fouling paints, however, is severely restricted currently because organotin compounds pollute sea.

There are also known anti-fouling paints for aquatic fouling organisms, using a rosin as a sea water-soluble resin. These paints, however, are far inferior to anti-fouling paints using an organotin copolymer, in anti-fouling property, film property, etc.

Further, there were proposed, as substitutes for organotin copolymer, an acrylic polymer into which a hydrolyzable group such as triorganosilyl group or the like has been introduced (U.S. Patent No. 4,593,055) and a graft copolymer of hydrolyzable polyester and vinyl polymer (JP-A-4-283278). These resins, however, have no satisfactory property for use in anti-fouling agent for aquatic fouling organisms.

### Disclosure of the Invention

The present invention has been made with main objects of providing a resin for use in anti-fouling agent for aquatic fouling organisms, which has high safety unlike the organotin compound and which has hydrolyzability similarly to the organotin copolymer, and an anti-fouling agent for aquatic fouling organisms, having a high anti-fouling property, obtained by using the above resin.

The present inventors made a study in order to achieve the above objects. As a result, the present inventors unexpectedly found out that an ester between an enol compound represented by the following formula (2): 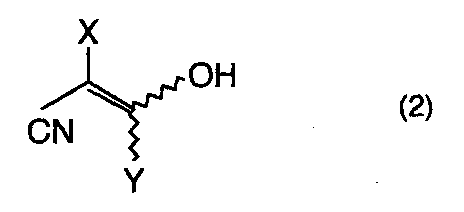 and (meth)acrylic acid is a novel compound which is hydrolyzable and, when the above ester is polymerized with at least one kind of unsaturated monomer selected from other unsaturated monomers having radical-polymerizable double bond, a copolymer is obtained which has appropriate hydrolyzability, etc. and which is suitably used as a resin for use in anti-fouling agent for aquatic fouling organisms. The present invention has been completed based on the above finding.

The above objects of the present invention have been achieved by providing the following inventions (1) to (4).
1. A compound used for production of a resin used in an anti-fouling agent for aquatic fouling organisms, said compound being represented by the following general formula (1): 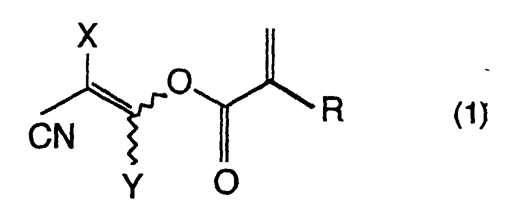 wherein R is a hydrogen atom or a methyl group; Y is a lower alkyl group or a phenyl group which may be substituted with halogen atom and/or lower alkyl group; and X is a phenyl group which may be substituted with halogen atom and/or lower alkyl group, a 2-thiazolyl group which may be substituted with lower alkyl group; a 2-thiazolyl group having, at the 4-position, a phenyl group which may be substituted with halogen atom and/or lower alkyl group; or a 2-benzothiazolyl group which may be substituted with halogen atom and/or lower alkyl group.
2. A resin used in an anti-fouling agent for aquatic fouling organisms, which is obtained by polymerizing at least one kind of compound represented by the general formula (1) and at least one kind of unsaturated monomer selected from other unsaturated monomers having radical-polymerizable double bond.
3. A process for producing a resin used in an anti-fouling agent for aquatic fouling organisms, which comprises polymerizing at least one kind of compound represented by the general formula (1) and at least one kind of unsaturated monomer selected from other unsaturated monomers having radical-polymeri zable double bond.
4. An anti-fouling agent for aquatic fouling organisms, comprising a resin obtained by polymerizing at least one kind of compound represented by the general formula (1) and at least one kind of unsaturated monomer selected from other unsaturated monomers having radical-polymerizable double bond.

### Best Mode for Carrying Out the Invention

Detailed description is made below on the compound used in production of the present resin for use in anti-fouling agent for aquatic fouling organisms; said resin for use in anti-fouling agent for aquatic fouling organisms; the process for production of said resin; and the anti-fouling agent for aquatic fouling organisms using said resin.

Description is made first on the compound used in production of the present resin for use in anti-fouling agent for aquatic fouling organisms.

The compound of the present invention can be an enol ester represented by the general formula (1) [the enol ester is expressed as "enol ester (1)" in some cases, the same applies to other compounds]. In the general formula (1), R is a hydrogen atom or a methyl group; Y is a lower alkyl group (the lower alkyl group refers to a C₁₋₈ alkyl group which may have a straight chain, a branched chain and/or an alicyclic structure, and specifically includes, for example, methyl group, ethyl group, n-propyl group, isopropyl group, n-butyl group, isobutyl group, tert-butyl group, sec-butyl group, n-pentyl group, cyclopentyl group, n-hexyl group, cyclohexyl group, n-heptyl group and n-octyl group; hereinafter, the lower alkyl group has the same definition), or a phenyl group which may be substituted with halogen atom (the halogen atom includes chlorine atom, bromine atom, iodine atom and fluorine atom; hereinafter, the halogen atom has the same definition) and/or lower alkyl group; X is a phenyl group which may be substituted with halogen atom and/or lower alkyl group, a 2-thiazolyl group which may be substituted with lower alkyl group, a 2-thiazolyl group having, at the 4-position, a phenyl group which may be substituted with halogen atom and/or lower alkyl group; or a 2-benzothiazolyl group which may be substituted with halogen atom and/or lower alkyl group.

Specific examples of the compound (1) of the present invention are 1-(2-chlorophenyl)-2-cyano-2-(4-phenyl-2-thiazolyl)ethenyl=acrylate, 1-ethyl-2-cyano-2-(4-phenyl-2-thiazolyl)ethenyl=methacrylate, 1-phenyl-2-cyano-2-(4-tert-butyl-2-thiazolyl)ethenyl=acrylate, 1-n-propyl-2-cyano-2-(2-thiazolyl)ethenyl=methacrylate, 1-(2-bromophenyl)-2-cyano-2-[4-(2-methylphenyl)-2-thiazolyl]ethenyl=acrylate, 1-n-hexyl-2-cyano-2-[4-(4-chlorophenyl)-2-thiazolyl]ethenyl=methacrylate, 1-(2-chlorophenyl)-2-cyano-2-(4-chlorophenyl)ethenyl=acrylate, 1-(4-methylphenyl)-2-cyano-2-(4-methylphenyl)ethenyl=methacrylate, 1-n-propyl-2-cyano-2-phenylethenyl=acrylate, 1-methyl-2-cyano-2-(4-n-propyl-2-thiazolyl)ethenyl=methacrylate, 1-phenyl-2-cyano-2-(2-benzo-thiazolyl)ethenyl=acrylate, and 1-(2-chlorophenyl)-2-cyano-2-(5-chloro-2-benzothiazolyl)ethenyl=methacrylate.

Of the compounds (1) of the present invention, a compound (1) is preferred wherein Y is a phenyl group substituted with halogen atom and X is a 2-thiazolyl group having at the 4-position, a phenyl group (as substituent) which may be substituted with halogen atom and/or lower alkyl group. The reason is that the resin of the present invention for use in anti-fouling agent for aquatic fouling organisms, obtained from said compound (1) releases an enol compound showing excellent anti-fouling effect for barnacle.

The compound (1) of the present invention can be produced, for example, by, as shown in the following reaction formula, reacting a corresponding enol compound (2) with an acid halide (3) in the presence of a base in an inert solvent. (In the formula, R, X and Y have the same definitions as given previously; and A is a halogen atom.)

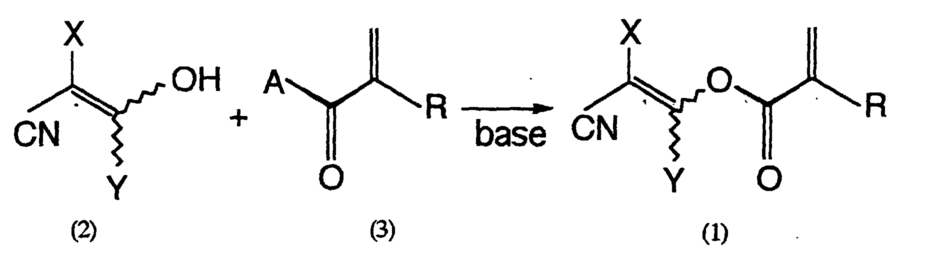

The enol compound (2) used in production of the compound (1) of the present invention can be produced by reacting corresponding phenylacetonitrile, 2-thiazolylacetonitrile or 2-benzothiazolylacetonitrile with an acid halide, or by subjecting said acetonitrile and an ester to Claisen condensation.

Specific examples of the enol compound (2) are 1-(2-chlorophenyl)-2-cyano-2-(4-phenyl-2-thiazolyl)ethenyl alcohol, 1-( 2-chlorophenyl)-2-cyano-2-(4-tert-butyl-2-thiazolyl)ethenyl alcohol, 1-(2-bromophenyl)-2-cyano-2-(4-(2-methylphenyl)-2-thiazolyl)ethenyl alcohol, 1-methyl-2-cyano-2-(4-phenyl-2-thiazolyl)ethenyl alcohol, 1-(2-chlorophenyl)-2-cyano-2-(4-chlorophenyl)ethenyl alcohol, 1-(4-chlorophenyl)-2-cyano-2-(4-methylphenyl)ethenyl alcohol, 1-n-propyl-2-cyano-2-(4-chlorophenyl)ethenyl alcohol, 1-ethyl-2-cyano-2-(4-tert-butyl-2-thiazolyl)ethenyl alcohol, 1-phenyl-2-cyano-2-(2-benzothiazolyl) ethenyl alcohol, and 1-(2-chlorophenyl)-2-cyano-2-(4-chloro-2-benzothiazolyl)ethenyl alcohol.

The acid halide (3) used in production of the compound (1) of the present invention can be a chloride, bromide or the like of acrylic acid or methacrylic acid. Specific examples thereof are acryloyl chloride and methacryloyl chloride. The amount of the acid halide (3) used can be ordinarily 1.0 to 1.5 moles, preferably 1.01 to 1.20 moles per mole of the enol compound (2).

As the base used in production of the compound (1) of the present invention, there can be mentioned, for example, inorganic bases such as sodium carbonate, potassium carbonate and the like, and organic bases such as triethylamine, pyridine and the like. The amount of the base used can be ordinarily 1.0 to 2.0 moles, preferably 1.2 to 1.7 moles per mole of the enol compound (2).

The solvent used in production of the compound (1) of the present invention can be any solvent inert to the raw materials, etc. used in the reaction. Specific examples thereof can be aromatic hydrocarbon type solvents such as toluene, xylene and the like; aliphatic hydrocarbon type solvents such as n-hexane, cyclohexane and the like; halogenated hydrocarbon type solvents such as chloroform, chlorobenzene and the like; and ketone type solvents such as acetone, methyl isobutyl ketone and the like. The amount of the solvent used can be any amount in which the reaction system can be stirred smoothly; however, the amount is ordinarily 0.3 to 3 ℓ, preferably 0.5 to 2 ℓ per mole of the enol compound (2).

The temperature of the reaction is 0°C to the refluxing temperature of the solvent, preferably 10 to 100°C.

Next, description is made on the present invention resin for use in anti-fouling agent for aquatic fouling organisms.

The present invention resin for use in anti-fouling agent for aquatic fouling organisms is hydrophobic per se. However, the resin undergoes hydrolysis in sea water, at the enol ester moiety; thereby, an enol compound represented by the general formula (2) is released; simultaneously therewith, the residual resin comes to have carboxyl group at the side chain and the carboxyl group forms a salt (e.g. sodium salt); as a result, the resin per se comes to have water solubility and show self-polishing property in sea water.

The present invention resin for use in anti-fouling agent for aquatic fouling organisms is a copolymer obtained by polymerizing at least one kind of compound represented by the general formula (1) and at least one kind of unsaturated monomer selected from other unsaturated monomers having radical-polymeri zable double bond (therefore, the resin is hereinafter referred to merely as "copolymer" in some cases). The configuration of components in copolymer may be any of random copolymerization, block copolymerization and graft copolymerization.

As the other unsaturated monomer having radical-polymerizable double bond (the other unsaturated monomer is hereinafter referred to as "other monomer" in some cases), there can be mentioned, for example, methyl=methacrylate, methyl=acrylate, butyl=acrylate, 2-ethylhexyl=acrylate, 2-hydroxy-ethyl=acrylate, dimethylaminoethyl=acrylate, acrylamide, acrylonitrile, acrylic acid, methacrylic acid, styrene, maleic anhydride, itaconic anhydride, vinyl acetate, butadiene, ethylene, vinyl chloride, vinylidene chloride, vinyl methyl ether and vinylpyrrolidone.

In the copolymer of the present invention, there is no particular restriction as to the proportions of the compound (1) of the present invention and the other monomer. However, the proportion of the present compound (1) is 10 to 90% by weight, preferably 20 to 80% by weight, more preferably 30 to 70% by weight. When the proportion of the present compound (1) is less than 10% by weight, the ester moiety of the resulting copolymer (the ester moiety undergoes hydrolysis in water) is too small; therefore, the copolymer, when hydrolyzed, has low water solubility and, when used, for example, as an anti-fouling agent for aquatic fouling organisms, shows no sufficient self-polishing property or anti-fouling property. When the proportion of the present compound (1) is more than 90% by weight and when the resulting copolymer is used, for example, as an anti-fouling paint for aquatic fouling organisms, the film formed has inferior properties, which may result in too high self-polishing property and short-term anti-fouling property.

There is no particular restriction as to the molecular weight of the copolymer of the present invention as long as the copolymer has film formability. However, the molecular weight is ordinarily 3,000 to 300,000, preferably 5,000 to 100,000 in terms of number-average molecular weight. Incidentally, in the present invention, the number-average molecular weight is a value measured by GPC (gel permeation chromatography) and then converted using a standard polystyrene calibration curve.

Next, description is made on the process for production of the copolymer of the present invention.

The copolymer of the present invention is produced by radical-polymerizing at least one kind of present compound (1) and at least one kind of unsaturated monomer (that is, the above-mentioned other monomer) selected from other unsaturated monomers having radical-polymerizable double bond.

As the polymerization process, there can be used any of bulk polymerization, solution polymerization, suspension polymerization and emulsion polymerization, all used in ordinary radical polymerization. However, when the present copolymer is used in an anti-fouling agent for aquatic fouling organisms and the agent is used as an anti-fouling paint, solution polymerization is preferred because the copolymer is used as a solution dissolved in an organic solvent. Hence, the solution polymerization is described below.

The polymerization is conducted by mixing the present compound of general formula (1), the other monomer and a polymerization initiator together in the presence of an organic solvent, or by dropping, into an organic solvent, part or all of the above-mentioned components to be polymerized. The latter dropping method is preferred because it is easy to control the heat of polymerization.

The proportions of the compound (1) of the present invention and the other monomer fed can be such that the proportion of the present compound (1) is 10 to 90% by weight, preferably 20 to 80% by weight, more preferably 30 to 70% by weight of the total weight of the above-mentioned components to be polymerized.

In the above polymerization, the organic solvent is used for dissolution of the present compound (1) as well as for control of the degree of polymerization of the copolymer of the present invention. As the organic solvent, there can be mentioned, for example, aromatic solvents such as toluene, xylene and the like; ketone type solvents such as methyl ethyl ketone, methyl isobutyl ketone and the like; ester type solvents such as ethyl acetate, butyl acetate and the like; alcohol type solvents such as methanol, ethanol and the like; and aprotic polar solvents such as dimethylformamide and the like.

The amount of the organic solvent used is 10 to 1,000 parts by weight, preferably 20 to 500 parts by weight, more preferably 30 to 300 parts by weight per 100 parts by weight of the total of the present compound (1) and the other monomer. When the amount is less than 10 parts by weight, the dissolution of the present compound (1) and the control of the degree of polymerization of the present copolymer are difficult. When the amount is more than 1,000 parts by weight, it is necessary to concentrate the solvent when the resulting copolymer is made into, for example, an anti-fouling paint for aquatic fouling organisms.

As the polymerization initiator used in the above polymerization, there can be mentioned, for example, azo compounds such as azobisisobutyronitrile and the like; and organic peroxides such as benzoyl peroxide, cumene hydroperoxide and the like. The amount of the polymerization initiator used can be 0.01 to 10 parts by weight per 100 parts by weight of the total of the present compound (1) and the other monomer.

There is no particular restriction as to the polymerization conditions used. However, the polymerization is conducted ordinarily in a nitrogen atmosphere at 40 to 120°C.

The copolymer of the present invention obtained as above is a random copolymer. It is also possible to obtain other type copolymer such as block copolymer, graft copolymer or the like, using the above-mentioned raw materials according to an ordinary process, and such a copolymer can be used in the present invention with no restriction. However, for use as a component resin in an anti-fouling agent for aquatic fouling organisms, a random copolymer is preferred because it is easiest to produce and yet exhibits a sufficient effect.

Next, description is made on the anti-fouling agent for aquatic fouling organisms using the copolymer of the present invention.

The anti-fouling agent for aquatic fouling organisms according to the present invention can be made into various appropriate forms such as paint, pellets, flakes, sheet and the like depending upon the purposes and applications, whereby the forms can be used in extensive applications requiring an anti-fouling agent for aquatic fouling organisms.

The anti-fouling agent for aquatic fouling organisms according to the present invention, when used in in-sea-water facilities (e.g. ship bottom, fishing net and buoy), underwater constructions and the portions of power station contacting with sea water (e.g. intake and heat exchanger), is ordinarily used as a paint containing the present copolymer as a resin; however, the present anti-fouling agent can also be used as a solid such as pellets, flakes, sheet or the like containing the present copolymer and can be used in a cooling water system utilizing sea water.

When the present anti-fouling agent for aquatic fouling organisms is used as a paint, there are mixed the present copolymer and, as necessary, a chemical having an anti-fouling activity for aquatic fouling organisms, a solvent, a plasticizer, other film-forming resin, a coloring pigment, an extender pigment and other components ordinarily used in paints; the resulting mixture is made into a paint by using a stirrer or an ordinary dispersing mixer such as three roll mill, sand mill or the like.

In the present anti-fouling agent for aquatic fouling organisms, the amount of the present copolymer used is not particularly restricted because the applications of the anti-fouling agent for aquatic fouling organisms are diversified (ship bottom, fishing net and underwater constructions) and the forms and formulations of the anti-fouling agent are also diversified. It is possible to use the present copolymer as it is; however, the present anti-fouling agent is ordinarily used in an appropriate form containing the present copolymer in an amount of 1 to 90% by weight.

The amount of the present copolymer used in such a form is determined appropriately depending upon the purpose, the kind of form, the method of use, etc. and can vary in a wide range as long as an intended form can be obtained. When, for example, a paint is obtained, the present copolymer is used in an amount of ordinarily 1 to 60% by weight, preferably 3 to 30% by weight; when pellets, flakes or a sheet is obtained, the present copolymer is used in an amount of ordinarily 1 to 90% by weight, preferably 2 to 80% by weight.

The above-mentioned other components used in an intended form can be those components ordinarily used in such a form. The kinds, combinations, amounts, etc. of other components are not restricted to those shown in the present specification (e.g., Examples shown later) and can be varied appropriately and widely depending upon, for example, the properties required for the form to be obtained.

The resin used in the anti-fouling agent for aquatic fouling organisms according to the present invention can be the present copolymer alone; however, it is possible to add other film-forming resin, for example, a synthetic resin (e.g. acrylic resin), a petroleum-based resin (e.g. coumarone resin), a thermoplastic synthetic rubber (e.g. styrene-butadiene rubber), a natural resin (e.g. rosin) or the like as long as the properties and hydrolyzability of the resulting film are satisfactory.

The anti-fouling agent for aquatic fouling organisms according to the present invention can further contain known compounds having an activity to aquatic fouling organisms, for example, copper compounds (e.g. cuprous oxide and copper rhodanide), zinc compounds (e.g. pyrithione zinc), boron compounds (e.g. triphenylboron=pyridinium) and other organic compounds. As these organic compounds, there can be mentioned, for example, substituted phenylmaleimides such as 2,3-dichloro-N-(2',6'-diethylphenyl)maleimide, N-(2,4,6-trichloropheny l)maleimide and the like; tetraethylthiuram disulfide; tetramethylthiuram disulfide; zinc dimethyldithiocarbamate; dizinc bis(dimethyldithio-carbamate) ethylene-bis (dithiocarbamate); tetrachloroisophthalo-nitrile; 2-thiocyanomet hylthiobenzothiazole; 3,4-dichlorophenyl-isothiocyanate; 4,5-dichloro-2-n-octyl-3(2H)isothiazolone; and 3-(3,4-dichlorophenyl )-1,1-dimethylurea. These compounds can be used singly or in combination of two or more kinds.

The anti-fouling agent for aquatic fouling organisms according to the present invention can further contain, as necessary, plasticizers such as dioctyl phthalate, chlorinated paraffin and the like; coloring pigments such as red iron oxide and the like; extender pigments such as talc, barium sulfate and the like; and various additives of amide type, silicon type, etc. ordinarily used in paints. When the present anti-fouling agent for aquatic fouling organisms is obtained as a form such as pellets, flakes or the like, there are as necessary added, for example, a hydrophilic resin solid at ordinary temperature (e.g. polyethylene glycol), a surfactant and a plasticizer, and the resulting mixture is subjected to compression molding or the like.

Next, description is made on the use of the present anti-fouling agent for aquatic fouling organisms.

The present anti-fouling agent for aquatic fouling organisms can be used by appropriately selecting one of the application methods ordinarily used, such as coating, impregnation, in-water placement and the like, depending upon the site, purpose and form of use of the anti-fouling agent. Specifically, when the present anti-fouling agent for aquatic fouling organisms is used in the form of, for example, paint, the anti-fouling agent can be used, for example, by coating on ship bottom, fishing net, underwater construction, intake, etc. When the present anti-fouling agent for aquatic fouling organisms is used for fishing net, the anti-fouling agent can also be used effectively by other method; that is, the anti-fouling agent is obtained as a paint or a solution and the fishing net is immersed in the paint or solution for coating and/or impregnation.

When the present anti-fouling agent for aquatic fouling organisms is used for intake, it is used as follows, for example. That is, the anti-fouling agent is obtained as pellets, flakes or a sheet and is used by a known method (e.g. suspension or attachment) appropriately selected so as to fit the actual situation of application site. The present anti-fouling agent for aquatic fouling organisms can also be used in and by application forms and methods other than mentioned above. For example, during the manufacturing process of fishing net or the like from a rope or a fiber material, the copolymer of the present invention can be incorporated into the rope or fiber material to allow the rope or fiber material per se to have an anti-fouling property for aquatic fouling organisms.

The present invention is hereinafter described more specifically by way of Examples. However, the present invention is in no way restricted by these Examples.

### Example 1 [Production of compound of general formula (1) wherein R is a hydrogen atom, Y is 2-chlorophenyl, and X is 4-phenyl-2-thiazolyl]

Into a 500-ml four-necked reaction flask provided with a stirrer, a thermometer, a Jimroth condenser and a dropping funnel were fed 67.7 g of 1-(2-chlorophenyl)-2-cyano-2-(4-phenyl-2-thiazolyl)ethenyl alcohol, 31.8 g of sodium carbonate and 250 g of acetone, followed by stirring at room temperature. Thereto was dropwise added 19.9 g of acryloyl chloride in 30 minutes at room temperature. After the completion of the dropwise addition, stirring was continued for 2 hours at the same temperature. The reaction mixture was poured into 1.5 ℓ of water, followed by stirring for 30 minutes. The resulting brown crystals were dissolved in 500 ml of toluene and the solution was washed with 300 ml of water three times. Toluene was reduced by concentration. The resulting light yellow crystals were collected by filtration and dried to obtain 42 g of 1-(2-chlorophenyl)-2-cyano-2-(4-phenyl-2-thiazolyl)ethenyl=acrylate (hereinafter abbreviated to TA).
Purity: 97.4% (by the total area method of high performance liquid chromatography (hereinafter abbreviated to HPLC)]
Melting point: 128 to 129°C

### Example 2 [Production of compound of general formula (1) wherein R is methyl, Y is 2-chlorophenyl, and X is 4-phenyl-2-thiazolyl]

Into a 500-ml four-necked reaction flask provided with a stirrer, a thermometer, a Jimroth condenser and a dropping funnel were fed 67.7 g of 1-(2-chlorophenyl)-2-cyano-2-(4-phenyl-2-thiazolyl)ethenyl alcohol, 31.8 g of sodium carbonate and 250 g of acetone, followed by stirring at room temperature. Thereto was dropwise added 23.0 g of methacryloyl chloride in 30 minutes at room temperature. After the completion of the dropwise addition, stirring was continued for 3 hours at the same temperature. The reaction mixture was poured into 1.5 ℓ of water, followed by stirring for 30 minutes. The resulting brown viscous material was dissolved in 500 ml of toluene and the solution was washed with 300 ml of water three times. Toluene was reduced by concentration. The resulting light yellow crystals were collected by filtration and dried to obtain 46.5g of 1-((2-chlorophenyl)-2-cyano-2-(4-phenyl-2-thiazolyl)ethenyl=methacrylate (hereinafter abbreviated to TMA).
Purity: 99.3% (HPLC, total area method)
Melting point: 125 to 127°C

### Example 3 [Production of compound of general formula (1) wherein R is methyl, Y is phenyl, and X is 4-chlorophenyl]

Into a 200-ml four-necked reaction flask provided with a stirrer, a thermometer, a Jimroth condenser and a dropping funnel were fed 20.4 g of 1-phenyl-2-cyano-2-(4-chloropheny l)ethenyl alcohol, 12.7 g of sodium carbonate and 80 g of acetone, followed by stirring at room temperature. Thereto was dropwise added 10.5 g of methacryloyl chloride in 10 minutes at room temperature. After the completion of the dropwise addition, stirring was continued for 3 hours at the same temperature. The reaction mixture was poured into 0.5 ℓ of water, followed by stirring for 10 minutes. A colorless oily material which separated from water, was extracted with 100 ml of toluene. The toluene solution was subjected to concentration and then cooled to 5°C. The separating colorless crystals were collected by filtration and dried to obtain 13.0 g of 1-phenyl-2-cyano-2-(4-chlorophenyl)ethenyl=methacrylate (hereinafter abbreviated to PCPMA).
Purity: 98.5% (HPLC, total area method)
Melting point: 78 to 80°C

### Example 4 [Production of compound of general formula (1) wherein R is methyl, Y is methyl, and X is 4-chlorophenyl]

Into a 200-ml four-necked reaction flask provided with a stirrer, a thermometer, a Jimroth condenser and a dropping funnel were fed 19.4 g of 1-methyl-2-cyano-2-(4-chloropheny l)ethenyl alcohol, 15.9 g of sodium carbonate and 50 g of acetone, followed by stirring at room temperature. Thereto was dropwise added 11.5 g of methacryloyl chloride in 30 minutes at room temperature. After the completion of the dropwise addition, stirring was continued for 3 hours at the same temperature. The reaction mixture was poured into 0.5 ℓ of water, followed by stirring for 10 minutes. A colorless oily material which separated from water, was extracted with 100 ml of toluene. The toluene solution was to concentrated to dryness to obtain 22.0 g of 1-methyl-2-cyano-2-(4-chlorophenyl)etheny l=methacrylate (hereinafter abbreviated to MCPMA).
Purity: 96.5% (HPLC, total area method)
Colorless viscous liquid

### Example 5 (Production of copolymer using TA)

Into a 100-ml four-necked reaction flask provided with a stirrer, a thermometer, a Jimroth condenser, a nitrogen-inlet tube and a dropping funnel were fed 15.7 g (0.04 mole) of TA and 20 g of toluene. The flask contents were kept at 80°C with stirring. Thereto was dropwise added, in a nitrogen current at the same temperature in 10 minutes, a solution obtained by dissolving 0.2 g of azobisisobutyronitrile (hereinafter abbreviated to AIBN) in 12.0 g (0.12 mole) of methyl methacrylate (hereinafter abbreviated to MMA). After the completion of the dropwise addition, stirring was continued for 2 hours at the same temperature. Then, a solution obtained by dissolving 0.04 g of AIBN in 10 g of toluene was dropwise added in 10 minutes at the same temperature. After the completion of the dropwise addition, stirring was, continued for 1 hour at the same temperature. 20 g of toluene and 30 g of methyl isobutyl ketone (hereinafter abbreviated to MIBK) were added, and the mixture was allowed to cool to obtain 95 g of a solution of a copolymer (hereinafter abbreviated to TAP-1). The copolymer had a number-average molecular weight of 30,000. Measurement of nonvolatile content was conducted (by a treatment of 100°C x 15 hours; in the following, measurement of nonvolatile content was conducted under the same conditions). The nonvolatile content was 28.5% and nearly agreed to the total weight of the monomers fed. The residual amount of TA was measured by HPLC and was 14.0%, which indicated that 86.0% of the TA fed was polymerized. From these results, the TAP-1 obtained was judged to be a random copolymer having a TA:MMA weight ratio of 13.5:12.0. The TAP-1 was measured for viscosity at 25°C using a B type viscometer (in the following Examples, viscosity measurement was made by the same method), which was 330 cp.

### Example 6 (Production of copolymer using TMA)

Into a 100-ml four-necked reaction flask provided with a stirrer, a thermometer, a Jimroth condenser, a nitrogen-inlet tube and a dropping funnel were fed 16.3 g (0.04 mole) of TMA and 20 g of toluene. The flask contents were kept at 80°C with stirring. Thereto was dropwise added, in a nitrogen current at the same temperature in 10 minutes, a solution obtained by dissolving 0.2 g of AIBN in 12.0 g (0.12 mole) of MMA. After the completion of the dropwise addition, stirring was continued for 1 hour at the same temperature. Then, a solution obtained by dissolving 0.04 g of AIBN in 10 g of toluene was dropwise added in 10 minutes at the same temperature. After the completion of the dropwise addition, stirring was continued for 1 hour at the same temperature. 40 g of MIBK was added, and the mixture was allowed to cool to obtain 88 g of a solution of a copolymer (hereinafter abbreviated to TMAP-1). The copolymer had a number-average molecular weight of 22,000. Measurement of nonvolatile content was conducted. The nonvolatile content was 32.0% and nearly agreed to the total weight of the monomers fed. The residual amount of TMA was measured by HPLC and was 3.8%, which indicated that 96.2% of the TMA fed was polymerized. From these results, the TMAP-1 obtained was judged to be a random copolymer having a TMA:MMA weight ratio of 15.7:12.0. The TMAP-1 was measured for viscosity at 25°C, which was 550 cp.

### Example 7 (Production of copolymer using PCPMA)

Into a 100-ml four-necked reaction flask provided with a stirrer, a thermometer, a Jimroth condenser, a nitrogen-inlet tube and a dropping funnel were fed 9.7 g (0.03 mole) of PCPMA and 15 g of toluene. The flask contents were kept at 80°C with stirring. Thereto was dropwise added, in a nitrogen current at the same temperature in 10 minutes, a solution obtained by dissolving 0.18g of AIBN in 9.0 g (0.09 mole) of MMA. After the completion of the dropwise addition, stirring was continued for 2 hours at the same temperature. Then, a solution obtained by dissolving 0.04 g of AIBN in 10 g of toluene was dropwise added in 10 minutes at the same temperature. After the completion of the dropwise addition, stirring was continued for 1 hour at the same temperature. 15 g of toluene and 5 g of MIBK were added, and the mixture was allowed to cool to obtain 54 g of a solution of a copolymer (hereinafter abbreviated to PCPMAP). The copolymer had a number-average molecular weight of 15,000. Measurement of nonvolatile content was conducted. The nonvolatile content was 35.2% and nearly agreed to the total weight of the monomers fed. The residual amount of PCPMA was measured by HPLC and was 2.2%, which indicated that 97.8% of the PCPMA fed was polymerized. From these results, the PCPMAP obtained was judged to be a random copolymer having a PCPMA:MMA weight ratio of 9.5:9.0. The PCPMAP was measured for viscosity at 25°C, which was 250 cp.

### Example 8 Production of copolymer using MCPMA)

Into a 100-ml four-necked reaction flask provided with a stirrer, a thermometer, a Jimroth condenser, a nitrogen-inlet tube and a dropping funnel was fed 30 g of toluene. The toluene was kept at 80°C with stirring. Thereto were dropwise added, in a nitrogen current at the same temperature in 30 minutes, 10.5 g (0.04 mole) of MCPMA, 12.0 g (0.12 mole) of MMA and a solution obtained by dissolving 0.2 g of AIBN in 15 g of toluene. After the completion of the dropwise addition, stirring was continued for 3 hours at the same temperature. Then, a solution obtained by dissolving 0.04 g of AIBN in 10 g of toluene was dropwise added in 10 minutes at the same temperature. After the completion of the dropwise addition, stirring was continued for 1 hour at the same temperature. The reaction mixture was allowed to cool to obtain 67 g of a solution of a copolymer (hereinafter abbreviated to MCPMAP). The copolymer had a number-average molecular weight of 18,000. Measurement of nonvolatile content was conducted. The nonvolatile content was 33.0% and nearly agreed to the total weight of the monomers fed. The residual amount of MCPMA was measured by HPLC and was 3.8%, which indicated that 96.2% of the MCPMA fed was polymerized. From these results, the MCPMAP obtained was judged to be a random copolymer having a MCPMA:MMA weight ratio of 10.1:12.0. The MCPMAP was measured for viscosity at 25°C, which was 160 cp.

### Example 9 - 15

Various copolymer solutions were obtained using TA or TMA in a manner similar to those used in Examples 5 and 6. The unsaturated monomers used, the molar ratio thereof, the degree of polymerization thereof, the nonvolatile content of copolymer obtained, and the numberaverage molecular weight of copolymer are shown in Table 1.

**Table 1**

| Ex. | Symbol of copolymer solution | Molar ratio of unsaturated monomers fed | Degree of Polymerization of TA or TMA (%) | Non-volatile content (%) | Number-average molecular Weight |
|---|---|---|---|---|---|
| 9 | TAP-2 | TA:MMA:BA = 1:3:2 | 88.2 | 35.0 | 14000 |
| 10 | TAP-3 | TA:MMA:2HEMA = 1:4:1 | 93.0 | 27.9 | 13000 |
| 11 | TMAP-2 | TMA:MMA = 1:2 | 84.7 | 27.4 | 16000 |
| 12 | TMAP-3 | TMA:MMA = 1:4 | 90.5 | 31.4 | 13000 |
| 13 | TMAP-4 | TMA:MMA:2HEMA = 1:2:2 | 88.6 | 27.6 | 16000 |
| 14 | TMAP-5 | TMA:MMA:BMA:EHMA = 1:2:2:1 | 95.5 | 34.8 | 17000 |
| 15 | TMAP-6 | TMA:MMA:MAA = 1:5:0.5 | 92.8 | 29.7 | 21000 |

The symbols in Table 1 have the following meanings.
- TA:: 1-(2-chlorophenyl)-2-cyano-2-(4-phenyl-2-thiazolyl)ethenyl acrylate
- TMA:: 1-(2-chlorophenyl)-2-cyano-2-(4-phenyl-2-thiazolyl)ethenyl methacrylate
- MMA:: methyl methacrylate
- BA:: butyl acrylate
- 2HEMA:: 2-hydroxyethyl methacrylate
- BMA:: butyl methacrylate
- EHMA:: 2-ethylhexyl methacrylate
- MAA:: methacrylic acid

The copolymers obtained in Examples 9, 11 and 12 were judged to be each a random copolymer having the following component ratio.
Example 9 TA:MMA:BA = 13.8:12.0:10.2 (weight ratio)
Example 11 TMA:MMA = 13.8:8.0 (weight ratio)
Example 12 TMA:MMA = 14.8:16.0 (weight ratio)

The copolymers obtained in Examples 10, 13, 14 and 15 were judged to be each a random copolymer whose component ratio was the same as the ratio of monomers fed.

### Example 16 (Production of anti-fouling paint using TAP-1)

There were mixed 12 parts by weight (as solid content) of TAP-1, 4 parts by weight of 2,3-dichloro-N-(2',6'-diethylphenyl)maleimide, 4 parts by weight of 2,3-dichloro-N-(2'-methyl-6'-ethylphenyl)maleimide, 4 parts by weight of 2,6-diethylphenylmaleimide, 10 parts by weight of red iron oxide, 10 parts by weight of barite and 30 parts by weight of talc. The mixture was made into a paint using a paint shaker.

### Examples 17 to 26

Anti-fouling paints were produced using TAP-2, TAP-3, TMAP-1, TMAP-2, TMAP-3, TMAP-4, TMAP-5, PCPMAP and MCPMAP in the same manner as in Example 6.

### Measurement Examples 1 to 11

A polished mild steel plate of 15 cm in length and 7 cm in width was treated with a zinc-rich primer and a chlorinated rubber A/C. On the treated steel plate was spray-coated one of the paints produced in Examples 16 to 26 so as to give a film having a thickness of about 100 µm as dried. The resulting material was fitted to a rotary drum and allowed to rotate in a sea water at a circumferential speed of about 8.5 knots. After one month and two months of rotation, the thickness of film exhausted was measured using an electromagnetic thickness meter. The results are shown in Table 2.

**Table 2**

| Measurement Example | Corresponding Example | Symbol of copolymer solution | Lost film thickness (µm) | |
|---|---|---|---|---|
| | | | After 1 month | After 2 months |
| 1 | 16 | TAP-1 | 10 | 22 |
| 2 | 17 | TAP-2 | 5 | 15 |
| 3 | 18 | TAP-3 | 8 | 18 |
| 4 | 19 | TMAP-1 | 9 | 22 |
| 5 | 20 | TMAP-2 | 15 | 28 |
| 6 | 21 | TMAP-3 | 9 | 20 |
| 7 | 22 | TMAP-4 | 7 | 20 |
| 8 | 23 | TMAP-5 | 5 | 16 |
| 9 | 24 | TMAP-6 | 9 | 21 |
| 10 | 25 | PCPMAP | 5 | 11 |
| 11 | 26 | MCPMAP | 6 | 12 |

### Test Examples 1 to 11

10 g of one of the anti-fouling paints produced in Examples 16 to 26 was coated on a FRP plate of 30 cm in length and 10 cm in width. The coated plate was immersed in the sea water of Owase Bay of Mie Prefecture at a depth of 1.5 m from the water surface. For comparison, the FRP plate per se coated with no paint was also immersed in the same manner. These plates were periodically observed for anti-fouling effect. The results are shown in Table 3. The anti-fouling effect was expressed in the following 5 levels.
5: No attachment of periphyton
4: The area of attachment of fouling organisms is 20% or less.
3: The area of attachment of fouling organisms is 20% to 40%.
2: The area of attachment of fouling organisms is 40% to 50%.
1: The area of attachment of fouling organisms is 60% or more.

**Table 3**

| Test Example | Symbol of copolymer solution | Anti-fouling effect | | |
|---|---|---|---|---|
| | | After 1 month | After 3 months | After 6 months |
| 1 | TAP-1 | 5 | 5 | 5 |
| 2 | TAP-2 | 5 | 5 | 4 |
| 3 | TAP-3 | 5 | 5 | 4 |
| 4 | TMAP-1 | 5 | 5 | 5 |
| 5 | TMAP-2 | 5 | 5 | 5 |
| 6 | TMAP-3 | 5 | 5 | 5 |
| 7 | TMAP-4 | 5 | 5 | 5 |
| 8 | TMAP-5 | 5 | 5 | 4 |
| 9 | TMAP-6 | 5 | 5 | 4 |
| 10 | PCPMAP | 5 | 4 | 3 |
| 11 | MCPMAP | 5 | 4 | 3 |
| Comparison | - | 4 | 2 | 1 |

### Industrial Applicability

According to the present invention, there are provided a resin for use in an anti-fouling agent for aquatic fouling organisms used for prevention of attachment of aquatic organisms onto underwater construction, fishing net, ship bottom, etc.; a process for production of the resin; a compound used for production of the resin; and an anti-fouling agent for aquatic fouling organisms using the resin.

By using the present compound, there can be obtained the present resin for use in anti-fouling agent for aquatic fouling organisms, having a feature that the enol ester moiety of the compound is easily hydrolyzed in sea water and the remining copolymer becomes water-soluble. By using the present resin, there can be obtained, without using any organotin compound which causes sea pollution, the present anti-fouling agent for aquatic fouling organisms, having both good film polishing property and good anti-fouling property.

The present process for production of the present resin for use in anti-fouling agent for aquatic fouling organisms can easily produce said resin.

## Claims

1. A compound used for production of a resin used in an anti-fouling agent for aquatic fouling organisms, said compound being represented by the following general formula (1): 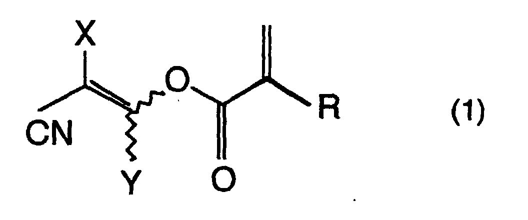 wherein R is a hydrogen atom or a methyl group; Y is a lower alkyl group or a phenyl group which may be substituted with halogen atom and/or lower alkyl group; and X is a phenyl group which may be substituted with halogen atom and/or lower alkyl group, a 2-thiazolyl group which may be substituted with lower alkyl group; a 2-thiazolyl group having, at the 4-position, a phenyl group which my be substituted with halogen atom and/or lower alkyl group; or a 2-benzothiazolyl group which may be substituted with halogen atom and/or lower alkyl group.

2. A resin used in an anti-fouling agent for aquatic fouling organisms, which is obtained by polymerizing at least one kind of compound represented by the general formula (1), set forth in Claim 1 and at least one kind of unsaturated monomer selected from other unsaturated monomers having radical-polymeri zable double bond.

3. A process for producing a resin used in an anti-fouling agent for aquatic fouling organisms, which is characterized by polymerizing at least one kind of compound represented by the general formula (1), set forth in Claim 1 and at least one kind of unsaturated monomer selected from other unsaturated monomers having radical-polymerizable double bond.

4. An anti-fouling agent for aquatic fouling organisms, which is characterized by containing a resin obtained by polymerizing at least one kind of compound represented by the general formula (1), set forth in Claim 1 and at least one kind of unsaturated monomer selected from other unsaturated monomers having radical-polymerizable double bond.
